# EUROPEAN PATENT SPECIFICATION

(11) **EP 4 117 544 B1**
(45) Date of publication and mention of the grant of the patent: **22.10.2025**
(21) Application number: 21716024.1
(22) Date of filing: 11.03.2021
(51) Int. Cl.: A61B 17/12, A61B 90/00, A61B 17/30

(54) **DEVICES FOR TISSUE LIGATION**
VORRICHTUNGEN ZUR GEWEBELIGATION
DISPOSITIFS DE LIGATURE DE TISSUS

(30) Priority: 12.03.2020 US 202062988479 P
(43) Date of publication of application: 18.01.2023
(73) Proprietor: Boston Scientific Scimed, Inc., Maple Grove, MN 55311 (US)
(72) Inventor: MOORE, Kyle P., Hopkinton, Massachusetts 01748 (US); WEBB, William D., San Francisco, California 94013 (US); FITZGERALD, Quinn, San Francisco, California 94013 (US); GREGORY, Julian, San Francisco, California 94013 (US)
(74) Representative: Vossius & Partner Patentanwälte Rechtsanwälte mbB
(86) International application number: PCT/US2021/021920
(87) International publication number: WO 2021/183770

(56) References cited:
- EP-A2- 1 147 744
- WO-A2-2016/134368
- US-A- 5 741 273
- US-A1- 2007 265 493
- US-A1- 2010 063 517
- US-A1- 2011 077 666
- US-A1- 2015 057 679
- US-B1- 6 482 213

## Description

### CROSS-REFERENCE TO RELATED APPLICATIONS

The present application is a non-provisional of, and claims the benefit of priority under 35 U.S.C. §119 to, U.S. Provisional Application Serial No. 62/988,479, filed March 12, 2020.

### FIELD

The present disclosure relates to medical ligating instruments and, more particularly, to devices and methods for tissue ligation using a tissue band dispenser.

### BACKGROUND

Hemorrhoids are an issue encountered by many adults, particularly as they age. One method to treat and/or remove hemorrhoids is ligation, wherein a constrictive element, such as an elastic band, surrounds and closes off tissue of the hemorrhoid(s). Over time, due to the lack of blood flow, the ligated hemorrhoid will eventually wither and fall off.

It can be difficult for a medical professional, such as a surgeon, to both position the ligation device at the ligation site and deploy elastic bands onto the target tissue. It is common practice for the physician to remove his or her guiding hand from the patient end of the ligation device in order to deploy a band, potentially losing position. It would therefore be desirable to have an apparatus which enables the surgeon to move the ligation device and simultaneously activate the release of the elastic bands using only one hand.

US 2007/265493 A1, EP1147744 A2 and US 2011/077666 A1 disclose ligation band applicators that are connected to an endoscope tip.

US 2010/063517 A1, WO 2016/134368 A2, US 2015/057679 A1 and US 5 741 273 A disclose ligation band applicators for delivery of a single ligation band. A plunger is provided, which provides upon retraction a suction force on the distal tip of the instrument.

### SUMMARY

The present invention is defined by the features of the independent claim. Embodiments of the invention are defined in the dependent claims.

The present disclosure in its various embodiments relates generally to medical devices, ligators, and methods for deploying ligation bands around a portion of tissue, such as a hemorrhoid. In one or more embodiments, a medical device may include a plunger moveable within a first tube, the first tube including a band along a first tube distal end. The medical device may further include a second tube for receiving the first tube, the second tube comprising a second tube proximal end opposite a second tube distal end, wherein the second tube distal end includes an engagement member operable to dispense the band from the first tube in response to axial movement of the first and second tubes relative to one another. In some embodiments the plunger may include a shaft extending between a plunger proximal end a plunger distal end, wherein a first channel extends through the plunger distal end, and a sealing member extending around the shaft, wherein the sealing member engages an inner surface of the first tube. In some embodiments, the first tube may include a first section, wherein the band extends around an exterior surface of the first section, and a second section connected to the first section, wherein the second section includes a user engagement feature, wherein the sealing member is disposed within the second section, and wherein a first diameter of the first section is less than a second diameter of the second section. In some embodiments, the second tube may include a distal section connected with a proximal section, wherein the proximal section extends around the second section of the first tube, and wherein the distal section extends around the first section of the first tube. In some embodiments, the engagement member may include a fixed end connected with the distal section of the second tube, and a free end extending axially from the fixed end, the free end comprising a flange extending radially towards the first tube. The flange may include a sloped surface operable to pass over the band as the first tube distal end and the second tube distal end move axially away to one another, and a planar surface operable to engage the band as the first tube distal end and the second tube distal end move axially towards one another. In some embodiments, the free end terminates proximal of a distal most point of the second tube distal end. In some embodiments, the medical device may further include one or more additional engagement members disposed about the second tube distal end, wherein the engagement member and the one or more additional engagement members are separated from one another by a slot. In some embodiments, the slot extends axially along the distal section of the second tube. In some embodiments, the medical device further includes an applicator engageable with a band loader, wherein band loader comprises the first tube and the second tube, The applicator may include a third tube surrounding the plunger, and a fourth tube surrounding the third tube, wherein the third tube extends within a second channel of the first tube to receive the band surrounding the first tube, and wherein the fourth tube includes a fourth tube distal end operable to dislodge and advance the band from the third tube as the third and fourth tubes move axially relative to one another.

In one or more embodiments, a ligator may include a plunger disposed within a first tube, the first tube including a plurality of bands surrounding a first tube distal end. The ligator may further include a second tube for receiving the first tube, the second tube comprising a second tube proximal end opposite a second tube distal end, wherein the second tube distal end includes a plurality of engagement members operable to engage and deploy each of the plurality of bands from the first tube in response to axial movement of the first tube relative to the second tube. In some embodiments, the plunger may include a shaft extending between a plunger proximal end a plunger distal end, and a sealing member extending around the shaft, wherein the sealing member engages an inner surface of the first tube to create a vacuum within the first tube. In some embodiments, the plurality of engagement members may include a fixed end connected with a distal section of the second tube, and a free end extending axially from the fixed end, wherein the free end comprises a flange extending radially towards the first tube. The flange may include a sloped surface operable to pass over the plurality of bands as the first tube distal end and the second tube distal end move axially away to one another, and a planar surface operable to engage the plurality of bands as the first tube distal end and the second tube distal end move axially towards one another. In some embodiments, the free end terminates proximal of a distal most point of the second tube distal end. In some embodiments, the ligator may further include a slot provided through the second tube distal end, the slot separating two adjacent engagement members of the plurality of engagement members.

In one or more embodiments, a method may include actuating a plunger disposed within a first tube to draw a target tissue into the first tube, the first tube including a band along a first tube distal end, and providing a second tube around the first tube. The second tube may include a second tube proximal end opposite a second tube distal end, wherein the second tube distal end includes an engagement member. The method may further include biasing the first and second tubes relative to one another to cause the engagement member to engage and deploy the band from the first tube distal end. In some embodiments, the method may further include biasing the second tube in a first axial direction towards a first tube proximal end of the first tube until a flange of the second tube passes over the band, and biasing the second tube in a second axial direction away from the first tube proximal end until a planar surface of the flange engages and deploys the band. In some embodiments, the method may further include biasing the second tube in the first axial direction towards the first tube proximal end of the first tube until the flange of the second tube passes over a second band, and biasing the second tube in the second axial direction away from the first tube proximal end until the planar surface of the flange engages and deploys the second band. In some embodiments, the method may further include providing a protection region at the second tube distal end, wherein the protection region is located between the engagement member and a distal most point of the second tube distal end to protect a free end of the engagement member as the second tube is inserted into a patient. In some embodiments, the method may further include engaging an applicator with a band loader, wherein the applicator includes a third tube surrounded by a fourth tube, and wherein the band loader comprises the first tube and the second tube. The method may further include biasing the second tube relative to the first tube to transfer the band from the first tube to the third tube. In some embodiments, the method may further include biasing the third and fourth tubes relative to one another to transfer the band from the third tube to a target tissue.

Various one or more of the features summarized above may be interchanged, exchanged, combined or substituted with or for other features summarized above, for use in connection with the medical systems and methods summarized above, and with respect to the embodiments described in greater detail below and embodiments otherwise within the scope of the present disclosure.

### BRIEF DESCRIPTION OF THE DRAWINGS

Non-limiting embodiments of the present disclosure are described by way of example with reference to the accompanying figures, which are not intended to be drawn to scale. In the figures, each identical or nearly identical component illustrated is typically represented by a single numeral. For purposes of clarity, not every component is labeled in every figure, nor is every component of each embodiment shown where illustration is not necessary to allow those of ordinary skill in the art to understand the disclosure. Furthermore, some of the figures include cross-sectional views in the form of "slices", or "near-sighted" cross-sectional views, omitting certain background lines or features otherwise visible in a "true" cross-sectional view, for illustrative clarity. In the figures:
**FIG. 1** is a perspective view of a medical device according to embodiments of the present disclosure;
**FIG. 2** is an exploded perspective view of the medical device of **FIG. 1** according to embodiments of the present disclosure;
**FIGS. 3A-3C** depict operation of the medical device of **FIG. 1** according to embodiments of the present disclosure;
**FIGS. 4A-4D** depict an approach for deploying one or more bands of the medical device of **FIG. 1** according to embodiments of the present disclosure;
**FIGS. 5A-5B** depict alternative medical devices according to embodiments of the present disclosure;
**FIG. 6** is a perspective view of a medical device according to embodiments of the present disclosure;
**FIG. 7** is an exploded perspective view of the medical device of **FIG. 6** according to embodiments of the present disclosure;
**FIGS. 8A-8C** depict operation of the medical device of **FIG. 6** according to embodiments of the present disclosure;
**FIGS. 9A-9D** depict an approach for deploying one or more bands of the medical device of **FIG. 6** according to embodiments of the present disclosure;
**FIGS. 10A-10B** depict alternative medical devices according to embodiments of the present disclosure;
**FIG. 11** is a perspective view of a medical device according to embodiments of the present disclosure;
**FIGS. 12A-12E** depict an approach for deploying one or more bands of the medical device of **FIG. 11** according to embodiments of the present disclosure;
**FIG. 13** is a perspective view of a plunger of the medical device of **FIG. 11** according to embodiments of the present disclosure; and
**FIG. 14** is a flow diagram of a method according to embodiments of the present disclosure.

### DETAILED DESCRIPTION

The present disclosure is not limited to the particular embodiments described herein. The terminology used herein is for the purpose of describing particular embodiments only, and is not intended to be limiting beyond the scope of the appended claims. Unless otherwise defined, all technical terms used herein have the same meaning as commonly understood by one of ordinary skill in the art to which the disclosure belongs.

Disclosed herein are medical devices, ligators, and methods for deploying ligation bands around a target tissue, such as a hemorrhoid. As described above, it would be desirable to have an apparatus that would more easily enable a medical professional to both maneuver a medical device (e.g., a ligator) into position around the hemorrhoid and activate the release of elastic bands therefrom. The medical devices, ligators, and methods of the present disclosure advantageously provide a simplified operator interface, including multiple, preloaded bands, which may be advanced and deployed/dispensed using one or more engagement members.

In some embodiments, the medical devices and ligators disclosed herein may include an applicator engageable with a band loader to transfer one or more bands onto the applicator. The band loader may include a first tube surrounded by a second tube, wherein the bands are disposed along an exterior of the first tube. Once the applicator and the band loader are coupled together, the bands may be transferred from the first tube to the applicator for subsequent deployment about the hemorrhoid.

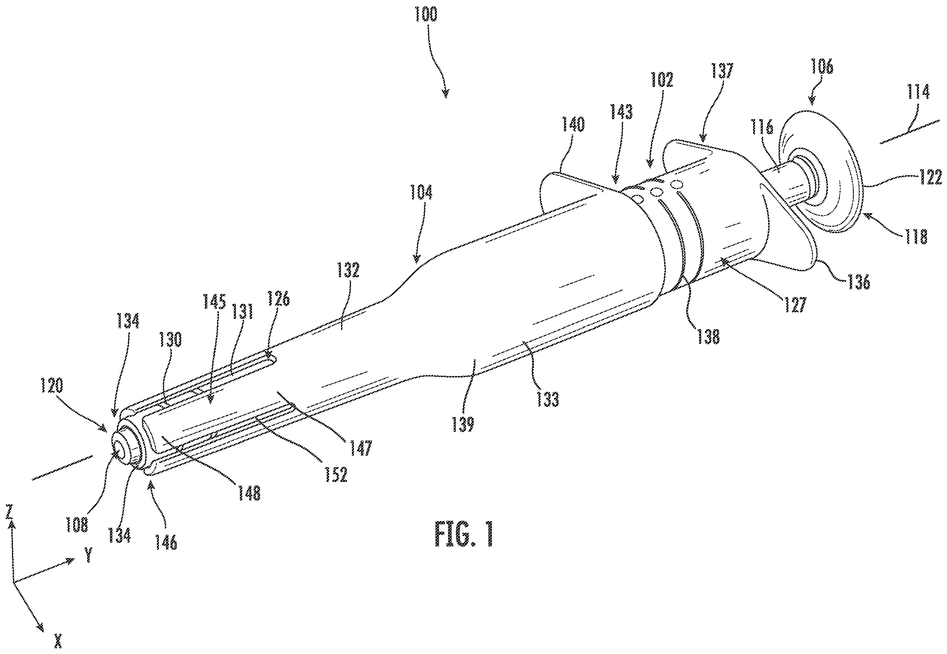

Turning now to **FIGS. 1-2****,** a medical device 100 (e.g., a ligator) according to embodiments of the disclosure will be described in greater detail. As shown, the medical device (hereinafter "device") 100 may include a first tube 102, a second tube 104, and a plunger 106. The first tube 102 may be a hollow elongate member for receiving the plunger therein 106, while the second tube 104 may be a hollow elongate member for receiving the first tube 102. The plunger 106 may define a first channel 108, the first tube 102 may define a second channel 110, and the second tube 104 may define a third channel 112. Although non-limiting, the plunger 106, the first tube 102, and the second tube 104 may be concentrically disposed about a central axis 114 extending through the device 100. In some embodiments, the plunger 106, the first tube 102, and the second tube 104 may be made from a flexible material, such as silicone, a thermoplastic elastomer including polyamide and polyether backbone blocks, polyurethane, etc., to allow for scope flexing. In other embodiments, one or more components of the device 100 may be made from a rigid material, such as polycarbonate, acrylonitrile butadiene styrene (ABS), etc., to provide a more direct positioning response.

The plunger 106 may include a rigid or semi-rigid shaft 116 extending between a plunger proximal end 118 and a plunger distal end 120. At the plunger proximal end 118 may be a plunger engagement component 122, such as a flange or a handle, which may be gripped by an operator to move the plunger 106 axially, relative to the first tube 102, along the central axis 114. At the plunger distal end 120 is a tip, which may be angled to prevent tissue to be pinched between an outer surface of the plunger distal end 120 and inner surface of the first tube 102. Although non-limiting, the angle may be greater than 25 degrees. One or more sealing members 124, such as a gasket, may surround the shaft 116. The sealing member 124 is operable to engage an inner surface of the first tube 102 to create an airtight seal at an interface therebetween.

The first tube 102 may include a first section 126 connected with a second section 127. As shown, a plurality of bands 130 are extendible around an exterior surface 131 of the first section 126. In some embodiments, the bands 130 may be partially seated within corresponding indentations (not shown). As will be described in greater detail below, the bands 130 may be elastic bands stretched to an expanded shape to fit around the exterior surface 131 of the first tube 102 and to maintain positioning until deployment. During operation, one or more of the bands 130 may be rolled or pushed off a first tube distal end 134 of the first tube 102, thereafter contracting about targeted tissue (not shown). Once deployed, the bands 130 may cut off blood flow to the targeted tissue such that the tissue will shrink, die, and eventually separate from surrounding tissue. The use of more than one band 130 may allow the device 100 to be repositioned for capturing additional tissue with sequential deployment of the plurality of bands 130 without having to withdraw the device 100 from the patient, thus allowing the clinician to more efficiently complete the procedure without the need to reload the device 100.

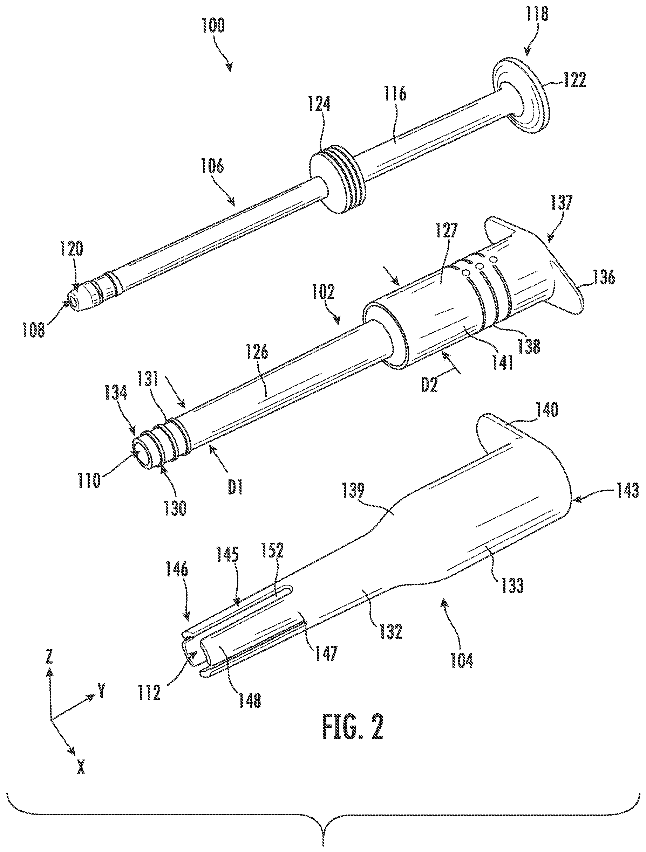

As further shown, the first tube 102 may include a user engagement feature 136, such as one or more tabs or flanges, extending radially from the second section 127. The user engagement feature 136 may be disposed at a first tube proximal end 137 of the first tube 102. In some embodiments, as better shown in **FIG. 2****,** a first diameter 'D1' of the first section 126 may be less than a second diameter 'D2' of the second section 127. Reducing the diameter of the first section 126 relative to the second section 127 may provide increased comfort to the patient. Once the device 100 is assembled, the plunger 106 may be positioned within the second channel 110, which extends through the first section 126 and the second section 127 of the first tube 102.

It will be appreciated that an inner diameter of the second section 127 is approximately equal to an outer diameter of the sealing member 124. In some embodiments, the inner diameter of the second section 127 may be slightly smaller than the outer diameter of the sealing member 124 to ensure an airtight seal therebetween.

As further shown, the second section 127 may include one or more ridges, indicators, or protrusions 138 along an exterior surface 141 thereof. The protrusions 138 may engage corresponding indentations, grooves, or detents (not shown) along an inner surface of second tube 104, or on a proximal end ridge. The protrusions 138 may provide visual, audible and/or tactile feedback to an operator of the device 100 regarding a position of the second tube 104 relative to the first tube 102, which may indicate, e.g., how many of the bands 130 have been deployed. In some embodiments, the inner surface of the second tube 104 may include a series of ridges or protrusions while the second section 127 includes the indentations or grooves. Furthermore, in some embodiments, the second section 127 and/or the inner surface of the second tube 104 may include one or more cantilevered cut-out tabs for engagement with an engagement feature on an opposite surface. It will be appreciated that any number or configuration of protrusions 138 is possible.

The second tube 104 may include a distal section 132 connected with a proximal section 133. The proximal section 133 may generally extend around the second section 127 of the first tube 102, and the distal section 132 may generally extend around the first section 126 of the first tube 102. Between the distal and proximal sections 132, 133 is a shoulder region 139. The shoulder region 139 may be gradually sloped and/or curved to increase comfort to the patient. As shown, a diameter of the distal section 132 may be less than a diameter of the proximal section 133. In some embodiments, the second tube 104 may include a biasing tab 140 extending radially from a second tube proximal end 143.

As further shown, the second tube 104 may include and one or more engagement members 145 extending axially at a second tube distal end 146. Each engagement member 145 may include a fixed end 147 integrally formed or connected with the distal section 132, and a free end 148 extending axially from the fixed end 147. As will be described in greater detail herein, the free end 148 of one or more engagement members 145 may include a flange extending radially towards the first tube 102 for engaging and deploying one or more bands. Adjacent engagement members 145 may be separated from one another by a gap or slot 152. As shown, each slot 152 may generally extend axially along the distal section 132 of the second tube 104. The slot 152 may provide flexibility to each of the engagement members 145, thus allowing the free end 148 to generally move radially relative to the central axis 114.

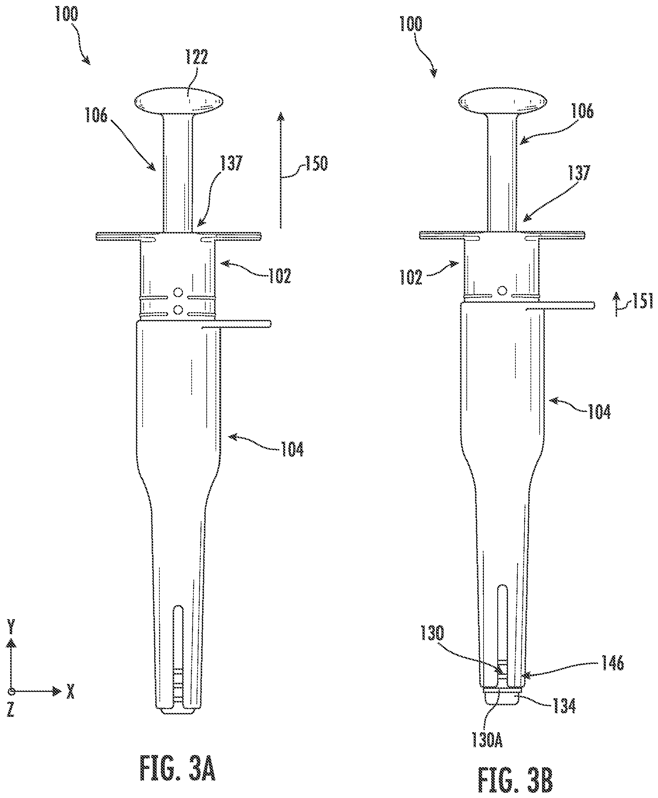

Turning now to **FIGS. 3A-3C****,** operation of the device 100 according to embodiments of the disclosure will be described. During use, an operator of the device 100 may target hemorrhoid tissue by, for example, looking through an endoscopic camera. In other embodiments, no camera is used. Suction may then be applied by biasing the plunger 106 to bring the target hemorrhoid tissue into the first tube 102. Specifically, as shown in **FIG. 3A****,** the plunger engagement component 122 of the plunger 106 may be moved axially away from the first tube proximal end 137 of the first tube 102 (e.g., along the y-direction), as shown by arrow 150. The sealing member 124 (**FIG. 2**) against the inner surface of the first tube 102 creates a vacuum chamber to draw the target hemorrhoid tissue into the device 100.

Next, as shown in **FIG. 3B** the second tube 104 may be biased relative to the first tube 102. For example, the second tube 104 may be drawn axially towards the first tube proximal end 137 of the first tube 102, as shown by arrow 151. As a result, the second tube distal end 146 moves away from the first tube distal end 134 to expose a distal most band 130A of the plurality of bands 130.

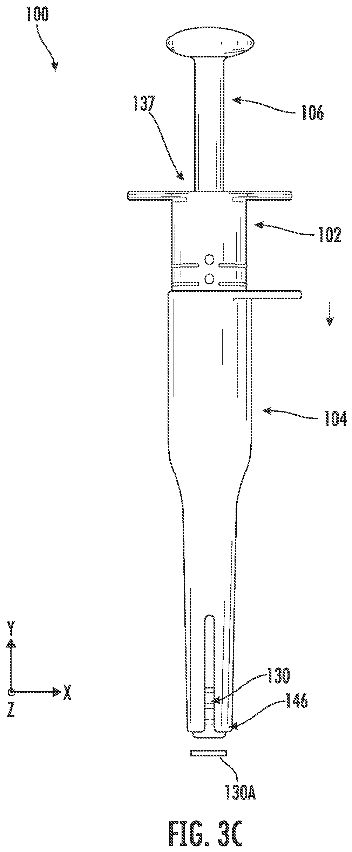

Next, as shown in **FIG. 3C****,** the second tube 104 may again be biased axially relative to the first tube 102. In this case, the second tube 104 moves away from the first tube proximal end 137 of the first tube 102, thus causing the second tube distal end 146 of the second tube 104 to engage and dislodge the distal most band 130A from the first tube 102. The distal most band 130A may then constrict around the target hemorrhoid tissue (not shown).

In some embodiments, suction may then be released by moving the plunger 106 towards the second tube distal end 146, and the proper positioning of the distal most band 130A confirmed, for example, by visualizing the site with the endoscopic camera. In other embodiments, suction may be maintained, and another band of the plurality of bands 130 deployed about the target hemorrhoid tissue.

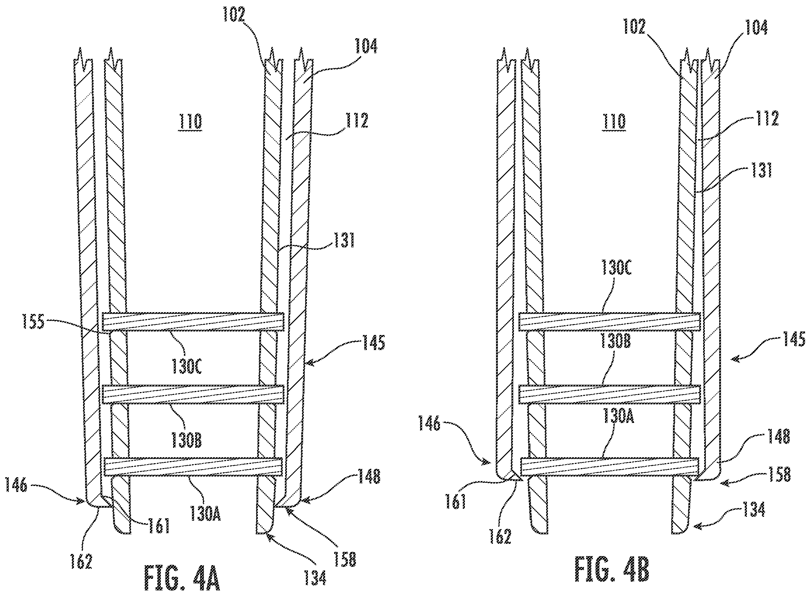

**FIGS. 4A-4D** further demonstrate deployment of the plurality of bands 130 from the device 100 according to embodiments of the present disclosure. As shown, the first tube 102 is disposed within the third channel 112 of the second tube 104. Although not shown, the plunger 106 may be disposed within the second channel 110 of the first tube 102. Each of the plurality of bands 130A-130C may be disposed in one or more corresponding indentations 155 of the first tube 102. In some embodiments, instead of indentations 155, a plurality of protrusions extending outwardly from the first tube 102 may separate the plurality of bands 130A-130C. Each engagement member 145 may include a flange 158 at the free end 148 thereof, wherein the flange 158 extends inwardly towards the first tube 102. In some embodiments, the flange 158 may be positioned at a distal most point of the second tube distal end 146.

In some embodiments, the flange 158 may include a sloped surface 161 extending to a point 162. As shown in **FIG. 4A****,** the point 162 may initially be engaged with the exterior surface 131 of the first tube 102. In an initial position, the flange 158 is positioned between the first tube distal end 134 and band 130A. As the second tube distal end 146 begins to move away from the first tube distal end 134, as demonstrated in **FIG. 4B****,** the sloped surface 161 passes over/along band 130A, causing the free end 148 of each engagement member 145 to flex radially away from the first tube 102.

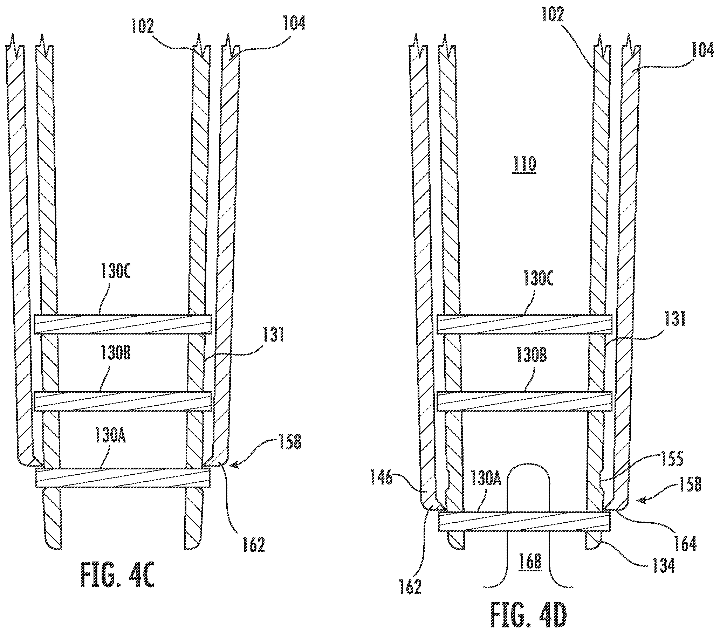

As shown in **FIG. 4C****,** the second tube 104 continues moving axially relative to the first tube 102 until the flange 158 passes band 130A. In this position, the flange 158 may engage or abut the exterior surface 131 of the first tube 102 in an area between bands 130A and 130B. In some embodiments, as the flange 158 re-engages with the exterior surface 131, an audible and/or tactile feedback to the operator of the device 100 regarding a position of the second tube 104 relative to the first tube 102. Once the operator hears an audible click, for example, the operator may stop moving the second tube 104 away from the first tube 102.

To begin removing band 130A from the first tube 102, as demonstrated in **FIG. 4D****,** the second tube distal end 146 may reverse direction and move towards the first tube distal end 134. The point 162, along with a planar surface 164 of the flange 158, may engage band 130A, removing it from the indentation 155. The point 162 may act as a wedge to dislodge the band 130A from the indentation 155. The planar surface 164 may continue to push the band 130A towards the first tube distal end 134 as the second tube 104 moves downwards. Eventually, the band 130A will slide beyond the first tube distal end 134 and around a target hemorrhoid tissue 168, which has been sucked into the second channel 110 of the first tube 102.

In some embodiments, once band 130A has been deployed, the second tube 104 may again move axially in an upward direction until the flange 158 passes band 130B and is positioned between bands 130B and 130C. Band 130B may then be deployed in a similar fashion to band 130A, followed by band 130C. It will be appreciated that a greater or fewer number of bands may be deployed in other embodiments.

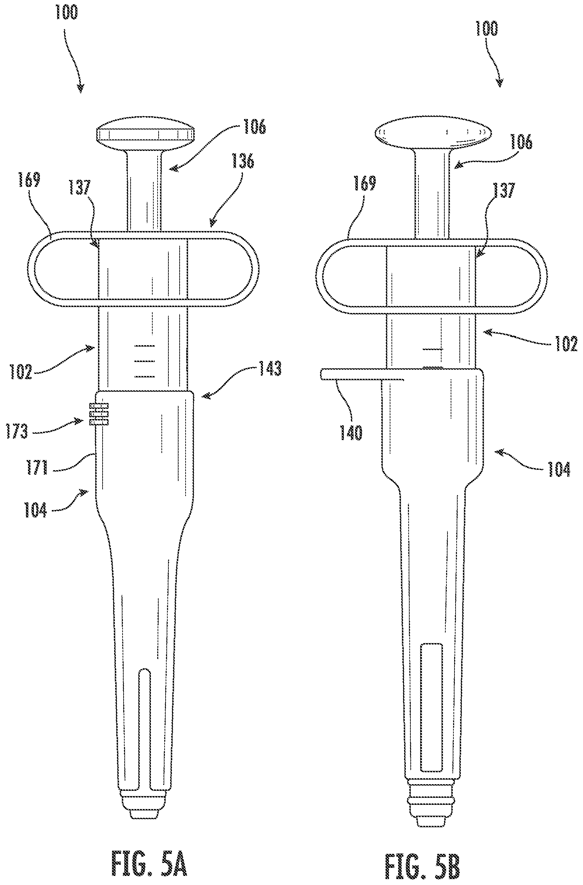

**FIGS. 5A** - **5B** show various non-limiting alternatives to the first and second tubes 102, 104 according to embodiments of the present disclosure. As shown, the user engagement feature 136 may be one or more loops 169 extending from or about the first tube proximal end 137 of the first tube 102. Each of the loops 169 may receive a finger of the operator to enhance operation of the device 100. As further shown in **FIG. 5A****,** the second tube 104 may include a set of grip elements 173 along an exterior surface 171 at the second tube proximal end 143. The set of grip elements 173 may replace or supplement the biasing tab 140, which is shown in the device 100 of **FIG. 5B****.**

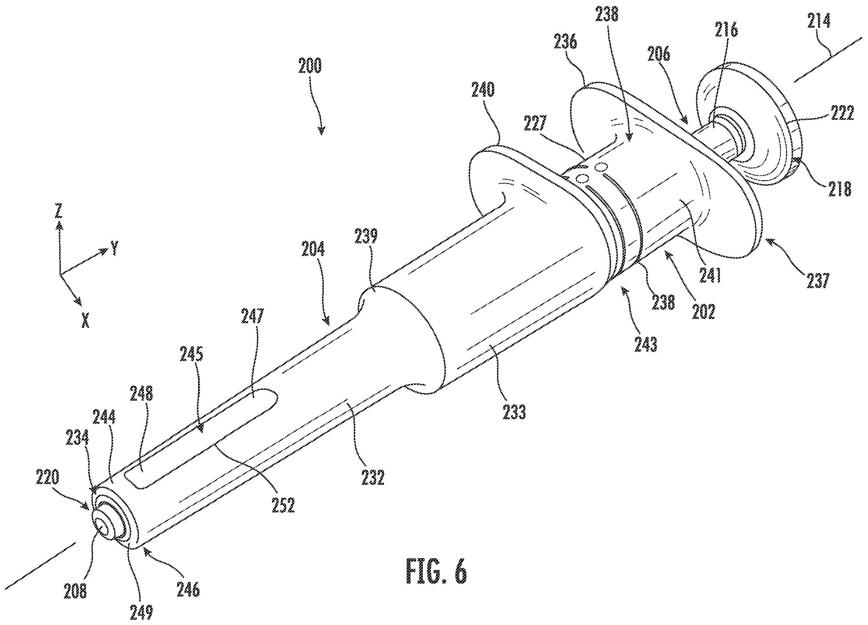

Turning now to **FIGS. 6-7****,** a medical device (e.g., a ligator) 200 according to embodiments of the disclosure will be described in greater detail. The medical device (hereinafter "device") 200 may be similar in many aspects to the device 100 described herein. As such, only certain aspects of the device 200 may hereinafter be discussed for the sake of brevity. As shown, the device 200 may include a first tube 202, a second tube 204, and a plunger 206. The first tube 202 may be a hollow elongate member containing the plunger therein 206, while the second tube 204 may be a hollow elongate member surrounding the first tube 202. The plunger 206 may define a first channel 208, the first tube 202 may define a second channel 210, and the second tube 204 may define a third channel 212. Although non-limiting, the plunger 206, the first tube 202, and the second tube 204 may be concentrically disposed about a central axis 214, which extends lengthwise through the device 200.

The plunger 206 may include a rigid or semi-rigid shaft 216 extending between a plunger proximal end 218 and a plunger distal end 220. At the plunger proximal end 218 may be a plunger engagement component 222, such as a flange or a handle, which may be gripped by an operator to move the plunger 206 axially relative to the first tube 202, e.g., along the y-direction. A sealing member 224, such as a gasket, may surround the shaft 216. The sealing member 224 is operable to engage an inner surface of the first tube 202 to create an airtight seal at an interface therebetween.

The first tube 202 may include a first section 226 connected with a second section 227, wherein a plurality of bands 230 extend around an exterior surface 231 of the first section 226. In some embodiments, the bands 230 may be partially seated within corresponding indentations of the first tube 202. As will be described in greater detail below, the bands 230 may be elastic bands stretched to an expanded shape to fit around the exterior surface 231 and to maintain positioning until deployment. During operation, one or more of the bands 230 may be rolled or pushed off a first tube distal end 234 of the first tube 202, thereafter contracting about targeted tissue (not shown). The use of more than one band 230 may allow the device 200 to be repositioned for capturing additional tissue with sequential deployment of the plurality of bands 230 without having to withdraw the device 200 from the patient.

Although non-limiting, the first tube 202 may include a user engagement feature 236, such as one or more tabs or flanges, extending radially from the second section 227. The user engagement feature 236 may be disposed at a first tube proximal end 237 of the first tube 202. Once the device 200 is assembled, the plunger 206 may be positioned within the second channel 210, which is defined by internal surfaces of the first section 226 and the second section 227 of the first tube 202.

As further shown, the second section 227 may include one or more indicators 238 along an exterior surface 241 thereof. The indicators 238 may provide visual, audible, and/or tactile feedback to an operator of the device 200 regarding a position of the second tube 204 relative to the first tube 202, which may further indicate, e.g., how many of the bands 230 have been deployed. It will be appreciated that any variety of indicators 238 may be used.

The second tube 204 may include a distal section 232 connected with a proximal section 233. The proximal section 233 may generally extend around the second section 227 of the first tube 202, wherein the second section 227 may include a biasing tab 240 extending radially from a second tube proximal end 243. Between the distal and proximal sections 232, 233 is a shoulder region 239. The shoulder region 239 may be gradually sloped and/or curved to increase comfort to the patient. For similar reasons, a diameter of the distal section 232 may be less than a diameter of the proximal section 233.

As further shown, the second tube 204 may include one or more engagement members 245 disposed along the distal section 232. In this embodiment, the engagement members 245 are positioned proximal of a second tube distal end 246 of the second tube 204. The engagement members 245 may include a fixed end 247 integrally formed or connected with the distal section 232, and a free end 248 extending axially from the fixed end 247. As will be described in greater detail herein, the free end 248 of one or more engagement members 245 may include a flange extending radially towards the first tube 202 to engage and deploy the plurality of bands 230. In some embodiments, the engagement members 245 may be surrounded by one or more slots 252 to provide flexibility to the free end 248.

As further shown, the second tube 204 may include a protection region 244 at the second tube distal end 246. The protection region 244 may include a substantially solid ring or cylinder provided between the free end 248 of the engagement members 245 and a distal most point 249 of the second tube distal end 246. That is, the protection region 244 may be devoid of any slots or gaps to increase rigidity of the second tube distal end 246. As such, the protection region 244 may prevent or reduce deflection of the free end 248 as the second tube distal end 246 is inserted into the patient.

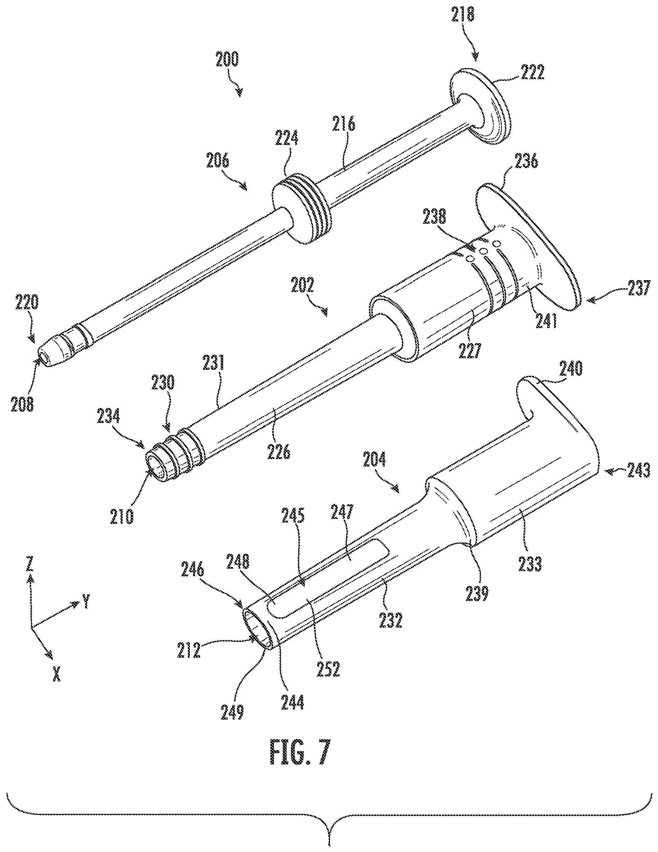

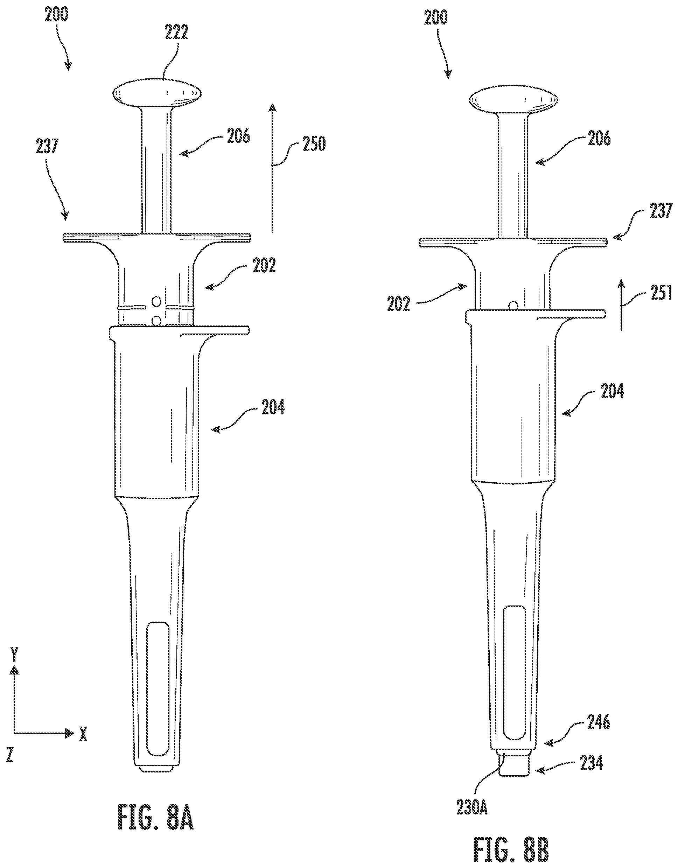

Turning now to **FIGS. 8A-8C****,** operation of the device 200 according to embodiments of the disclosure will be described. During use, an operator of the device 200 may target hemorrhoid tissue by, for example, looking through an endoscopic camera. Suction may then be applied by biasing the plunger 206 to create a vacuum and bring the target hemorrhoid tissue into the first tube 202. Specifically, as shown in **FIG. 8A****,** the plunger engagement component 222 of the plunger 206 may be moved axially away from the first tube proximal end 237 of the first tube 202 (e.g., along the y-direction), as shown by arrow 250. The sealing member 224 (**FIG. 7**) against the inner surface of the first tube 202 creates a vacuum chamber to draw the target hemorrhoid tissue into the device 200.

Next, as shown in **FIG. 8B** the second tube 204 may be biased relative to the first tube 202. For example, the second tube 204 may be drawn axially towards the first tube proximal end 237 of the first tube 202, as shown by arrow 251. Movement of the second tube distal end 246 of the second tube 204 relative to the first tube 202 exposes the first tube distal end 234 and a distal most band 230A of the plurality of bands 230.

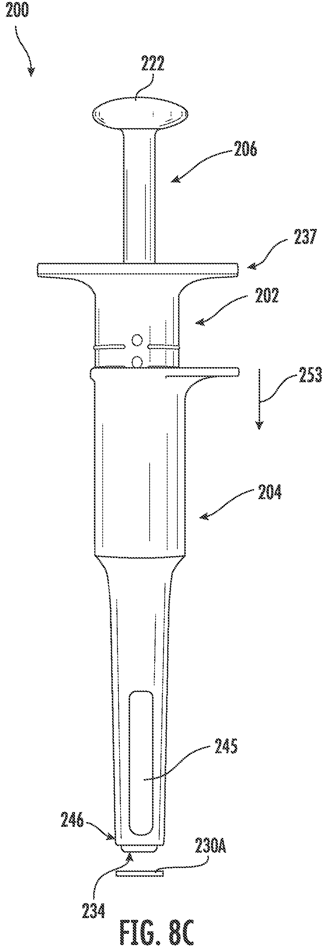

Next, as shown in **FIG. 8C****,** the second tube 204 may again be biased relative to the first tube 202. In this case, the second tube 204 may be moved axially away from the first tube proximal end 237 of the first tube 202, as shown by arrow 253, thus causing the engagement member 245 of the second tube 204 to engage and dislodge the distal most band 230A from the first tube distal end 234 of the first tube 202. The distal most band 230A may then constrict around the target hemorrhoid tissue (not shown).

In some embodiments, suction may then be released by moving the plunger 206 towards the second tube distal end 246, and the proper positioning of the distal most band 230A confirmed, for example, by visualizing the site with the endoscopic camera. In other embodiments, suction may be maintained, and another band of the plurality of bands 230 deployed about the target hemorrhoid tissue.

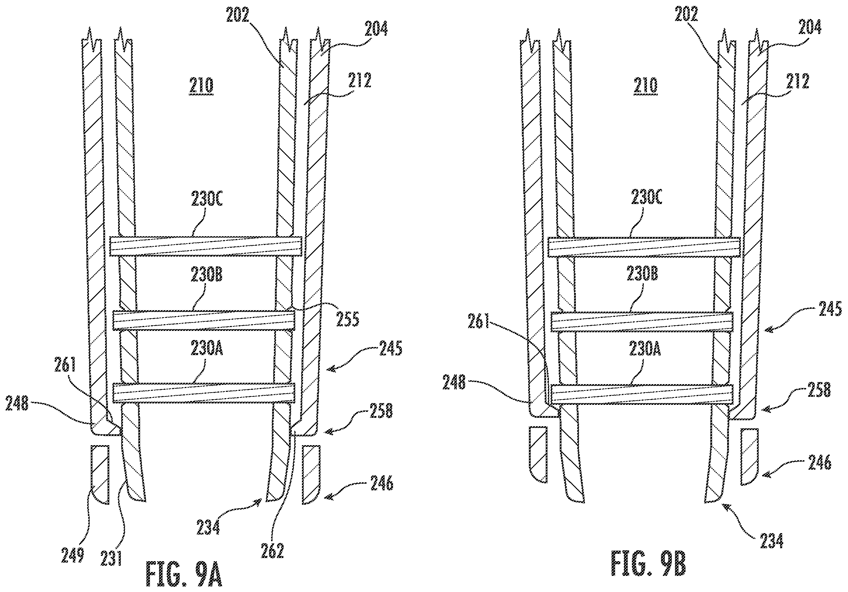

**FIGS. 9A-9D** further demonstrate deployment of the plurality of bands 230 from the device 200 according to embodiments of the present disclosure. As shown, the first tube 202 is disposed within the third channel 212 of the second tube 204. Although not shown, the plunger 206 may be disposed within the second channel 210. Each of the plurality of bands 230A-230C may be disposed in one or more corresponding indentations 255 of the first tube 202. Each engagement member 245 may include a flange 258 at the free end 248 thereof, wherein the flange 258 extends inwardly towards the first tube 202. As shown, the flange 258 of the free end 248 terminates proximal of the distal most point 249 of the second tube distal end 246.

In some embodiments, the flange 258 may include a sloped surface 261 extending to a point 262. The point 262 may initially be engaged with the exterior surface 231 of the first tube 202, as shown in **FIG. 9A****.** In this position of the device 200, the flange 258 is positioned between the first tube distal end 234 and band 230A. As the second tube distal end 246 begins to move away from the first tube distal end 234, as demonstrated in **FIG. 9B****,** the sloped surface 261 may engage band 230A, causing the free end 248 of each engagement member 245 to flex radially outward from the first tube 202.

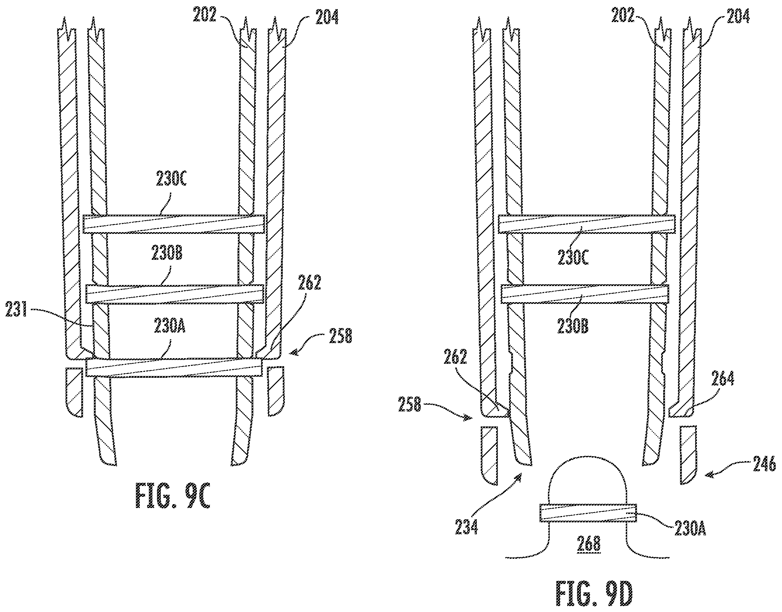

As shown in **FIG. 9C****,** the second tube 204 continues moving axially relative to the first tube 202 until the flange 258 passes band 230A. In this position, the flange 258 may engage or abut the exterior surface 231 of the first tube 202 in an area between bands 230A and 230B. To remove band 230A from the first tube 202, the second tube 204 may then reverse direction, moving towards the first tube distal end 234. The point 262, along with a planar surface 264 of the flange 258, may engage band 230A, removing it from the indentation 255, as shown in **FIG. 9D****.** The point 262 may act as a wedge to dislodge the band 230A from the indentation 255. The planar surface 264 may be used to push the band 230A towards the first tube distal end 234 as the second tube 204 moves downwards until the band 230A is deployed about a target hemorrhoid tissue 268. As shown, the band 230A constricts about the target hemorrhoid tissue 268 once deployed.

In some embodiments, once band 230A has been deployed, the second tube 204 may again move axially in an upward direction until the flange 258 passes band 230B and is positioned between bands 230B and 230C. Band 230B may then be deployed in a similar fashion to band 230A, followed by band 230C. It will be appreciated that a greater or fewer number of bands may be deployed in other embodiments.

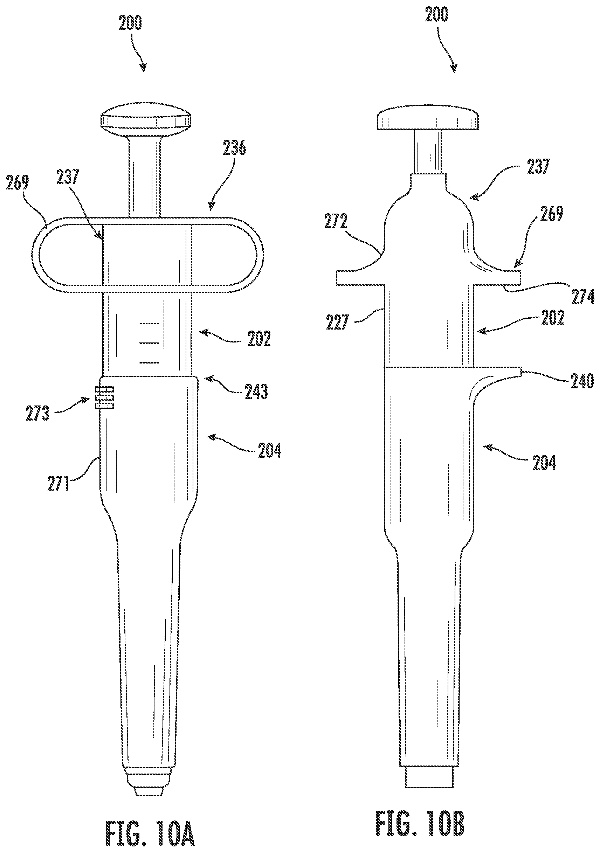

**FIGS. 10A - 10B** show various non-limiting alternatives to the first and second tubes 202, 204 according to embodiments of the present disclosure. As shown in **FIG. 10A****,** the user engagement feature 236 may include one or more loops 269 extending from or about the first tube proximal end 237 of the first tube 202. The second tube 204 may further include a set of grip elements 273 along an exterior surface 271 at the second tube proximal end 243. The set of grip elements 273 may be replace or supplement a biasing tab, such as the biasing tab 240 shown in **FIG. 10B****.** The device 200 of **FIG. 10B** may further include a pair of tabs 269 extending radially from the second section 227 of the first tube 202. Although non-limiting, the pair of tabs 269 may be positioned distal of the first tube proximal end 237. The pair of tabs 269 may include a sloped upper surface 272 and planar lower surface 274 for improved operator ergonomics.

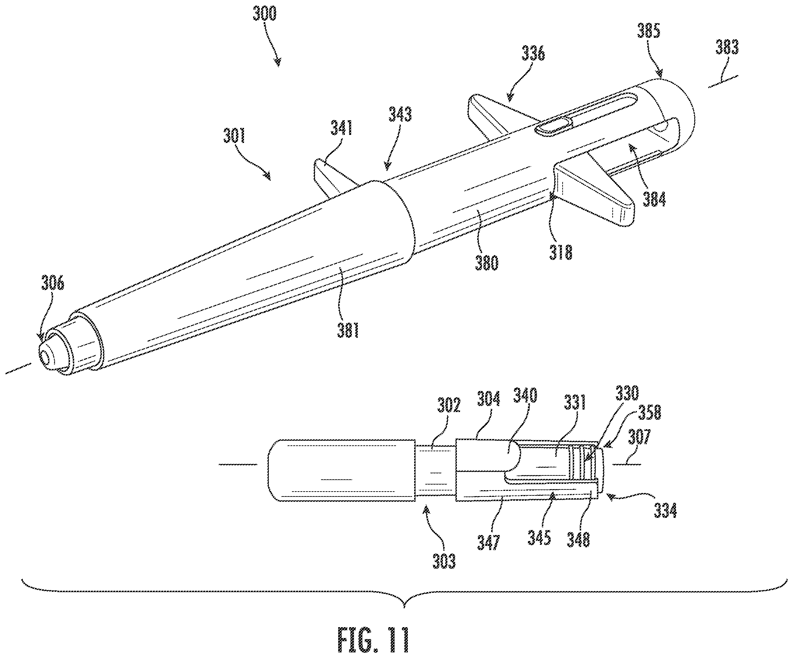

Turning to **FIG. 11****,** a medical device (e.g., a ligator) 300 according to embodiments of the disclosure will be described. As shown, the medical device (hereinafter "device") 300 may include an applicator 301 engageable with a band loader 303 to transfer one or more bands 330 onto the applicator 301. The band loader 303 may include a first tube 302 surrounded by a second tube 304. The first tube 302 and the second tube 304 may be hollow elongate members concentrically disposed about a band loader axis 307. The first tube 302 may include a first tube distal end 334 containing the bands along an exterior surface 331 thereof.

As further shown, the second tube 304 may include a biasing surface 340, which enables an operator to move the second tube 304 axially relative to the first tube 302, e.g., along the band loader axis 307, to deploy the bands 330. In some embodiments, the biasing surface 340 is textured and/or includes one or more gripping elements (e.g., ridges) for enhanced engagement with a thumb of the operator. The second tube 304 may further include one or more engagement members 345 including a fixed end 347 opposite a free end 348. As will be described in greater detail herein, the free end 348 of one or more engagement members 345 may include a flange 358 extending radially towards the first tube 302 to engage and dislodge the bands 330. In some embodiments, each of the engagement members 345 may be separated from one another by a gap to provide radial flexibility thereto.

The applicator 301 may include a third tube 380 surrounding a plunger 306, and a fourth tube 381 surrounding the third tube 380. As shown, the plunger 306, the third tube 380, and the fourth tube 381 may be hollow elongate members concentrically disposed about an applicator axis 383. The plunger 306 may include a user engagement feature 336, such as one or more tabs or flanges, extending radially from a plunger proximal end 318. The user engagement feature 336 enables the plunger 306 to be biased, along the applicator axis 383, relative to the third tube 380. As shown, the user engagement feature 336 of the plunger 306 may slide within an opening or slot 384 at a third tube proximal end 385 of the third tube 380. In some embodiments, the fourth tube 381 may include a biasing tab 341 extending radially from a fourth tube proximal end 343, the biasing tab 341 permitting actuation of the fourth tube 381 relative to the third tube 380.

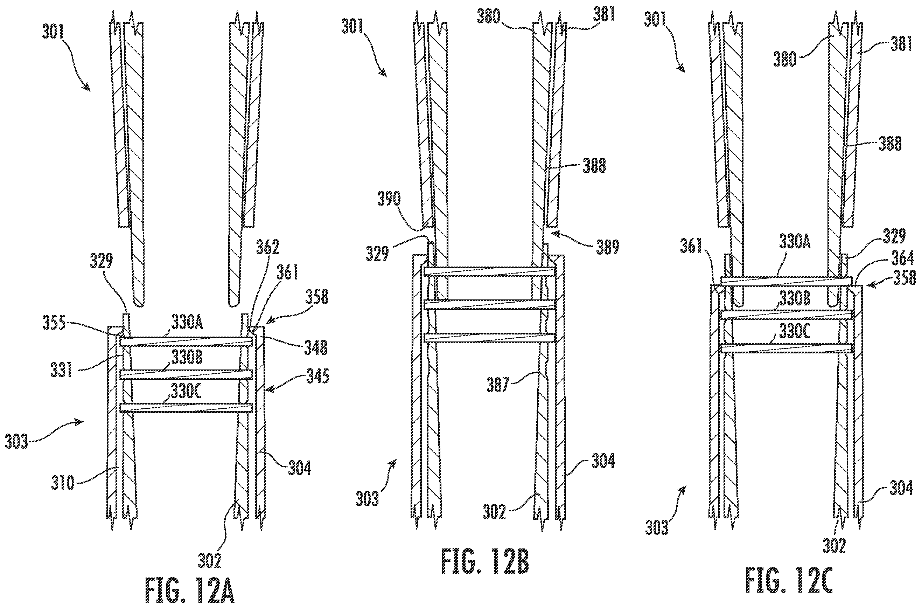

**FIGS. 12A-12E** demonstrate transfer of the plurality of bands 330 from the band loader 303 to the applicator 301 according to embodiments of the present disclosure. As shown, the band loader 303 includes the first tube 302 disposed within a second channel 310 of the second tube 304. Each of the plurality of bands 330A-330C may be disposed in one or more corresponding indentations of the first tube 302. Each engagement member 345 may include the flange 358 at the free end 348 thereof, wherein the flange 358 extends inwardly towards the first tube 302. As shown, flange 358 may be positioned between band 330A and a distal most point 329 of the first tube 302. In some embodiments, the flange 358 may include a sloped surface 361 extending to a point 362, wherein the point 362 is engaged with the exterior surface 331 of the first tube 302. The applicator 301 and the band loader 303 may initially be separated from one another, as shown in **FIG. 12A****.**

When the applicator 301 and the band loader 303 are brought into engagement, as demonstrated in **FIG. 12B****,** the first tube 302 may be positioned over the third tube 380. For example, an internal surface 387 of the first tube 302 may be brought adjacent to, and/or into physical contact with, an exterior surface 388 of the third tube 380. As shown, a gap 389 may be provided between the distal most point 329 of the first tube 302 and a distal most point 390 of the fourth tube 381 to allow receipt of the plurality of bands 330A-330C about the third tube 380 upon deployment.

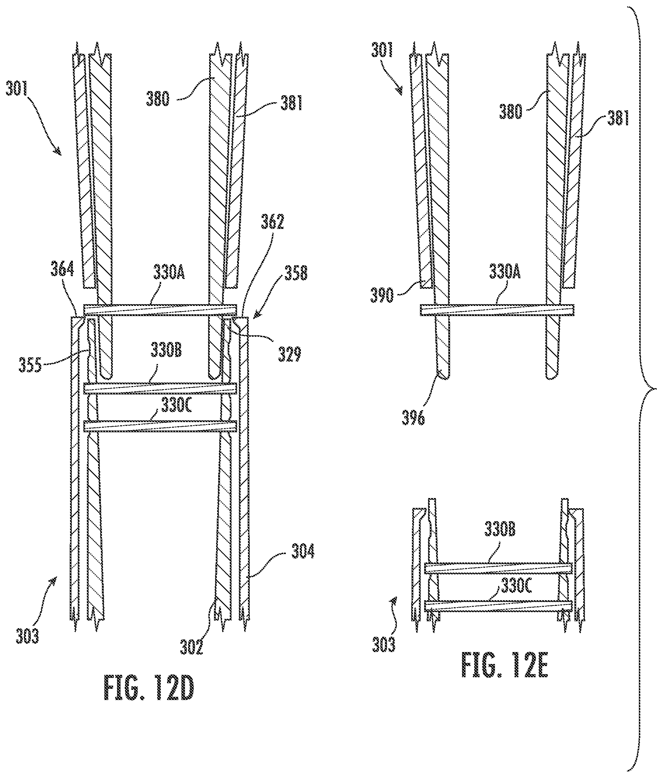

Next, as shown in **FIG. 12C****,** the second tube 304 may be retracted relative to the first tube 302 to bring the flange 358 between bands 330A and 330B. The sloped surface 361 permits the flange 358 to more easily pass over the band 330A. The second tube 304 may then be moved towards the distal most point 329 of the first tube 302 until a planar surface 364 of the flange 358 engages the band 330A. As shown in **FIG. 12D****,** the point 362 and the planar surface 364 may remove band 330A from the indentation 355, pushing the band 330A towards the applicator 301 until the band 330A passes beyond the distal most point 329 of the first tube 302 and onto the third tube 380.

As shown in **FIG. 12E****,** once band 330A has been deployed about the third tube 380, the band loader 303 may disengage from the applicator 301. Although not shown, the third tube 380 of the applicator 301 may then be brought into position about a target hemorrhoid tissue. The band 330A may be deployed by biasing the distal most point 390 of the fourth tube 381 against the band 330A until the band 330A is slid or pushed beyond a fourth tube distal end 396 of the third tube 380 and onto the target hemorrhoid tissue. Although not shown, the fourth tube 381 may include one or more flanges, such as flanges 158, 258 described above, configured to engage and deploy the band 330A.

In some embodiments, the band loader 303 and the applicator 301 may remain engaged until all of the bands 330A-330C are deployed onto the third tube 380. It will be appreciated that bands 330B and 330C may be deployed in a similar fashion to band 330A. It will further be appreciated that a greater or fewer number of bands may be deployed in other embodiments.

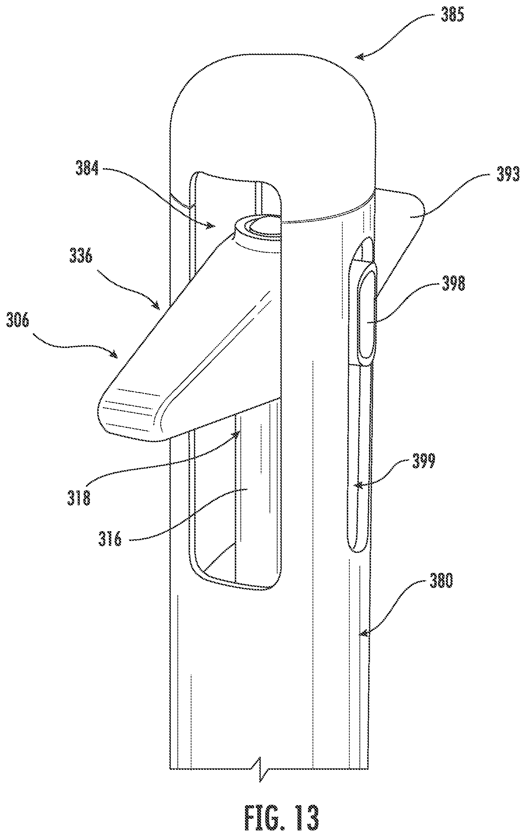

Turning now to **FIG. 13****,** the plunger 306 of the device 300 according to embodiments of the present disclosure will be described in greater detail. As shown, the plunger 306 may include a rigid or semi-rigid shaft 316 extending within the third tube 380. At the plunger proximal end 318 is the user engagement feature 336, such as one or more tabs or flanges 393, extending radially therefrom. The flanges 393 may extend through the opening 384 at the third tube proximal end 385 of the third tube 380. Although not shown, a sealing member, such as a gasket, may surround the shaft 316, engaged with an inner surface of the third tube 380 to create an airtight seal at an interface therebetween.

During use, the third tube proximal end 385 of the third tube 380 may be positioned within a palm of the operator, while pulling of the user engagement feature 336 towards the third tube proximal end 385 with his/her fingers increases a vacuum pressure within the third tube 380. In this way, the device 300 permits one-handed operation. It will be appreciated that a similar engagement feature may be incorporated into device 100 and 200, described herein, in place of plunger engagement components 122 and 222, respectively.

Once the target hemorrhoid tissue is drawn into the third tube 380, and the bands are deployed from the third tube 380, suction created by the plunger 306 and the third tube 380 may be released. In some embodiments, the plunger 306 may include a relief component 398 actuatable by the operator. Although non-limiting, the relief component 398 may be a button for controlling a valve (not shown) within the plunger 306. Once the button is actuated, the valve is opened, which breaks the vacuum seal created by the plunger 306 and the third tube 380. In some embodiments, the relief component 398 may extend through a relief opening 399 of the third tube 380 for access by the operator.

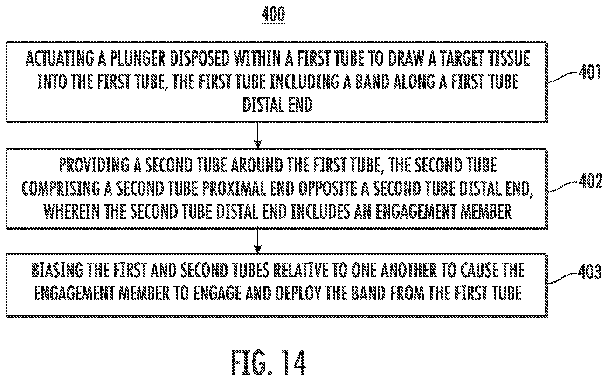

**FIG. 14** is a flow diagram of a method 400 according to embodiments of the present disclosure. At block 401, the method 400 may include actuating a plunger disposed within a first tube to draw a target tissue into the first tube, the first tube including a band along a first tube distal end. In some embodiments, to draw in the target tissue, a vacuum is created within the first tube using a sealing member of the plunger engaged with an inner surface of the first tube. In some embodiments, the sealing member is a circular gasket. In some embodiments, the plunger includes a rigid or semi-rigid shaft extending between a plunger proximal end and a plunger distal end. At the plunger proximal end may be a plunger engagement component, such as a flange or a handle, which may be gripped by an operator to move the plunger, e.g., in an axial direction, relative to the first tube.

At block 402, the method 400 may include providing a second tube around the first tube, the second tube comprising a second tube proximal end opposite a second tube distal end, wherein the second tube distal end includes an engagement member. In some embodiments, the plunger, the first tube, and the second tube may be concentrically disposed about a central axis extending through the device.

At block 403, the method 400 may include biasing the first and second tubes relative to one another to cause the engagement member to engage and deploy the band from the first tube. In some embodiments, the method may include biasing the second tube in a first axial direction towards a first tube proximal end of the first tube until a flange of the second tube passes over the band, and then biasing the second tube in a second axial direction away from the first tube proximal end until a planar surface of the flange engages the band. The second tube may continue to be biased until the band is deployed beyond a first tube proximal end of the first tube. In some embodiments, the band may be deployed about the target tissue. In some embodiments, the band may be deployed about a shaft of an applicator, which may then be inserted within a patient.

In some embodiments, the first and second tubes are part of a band loader operable with the applicator to load one or more bands onto a third tube of the applicator. During use, the applicator and the band loader may be brought together such that an internal surface of the first tube is brought into physical contact with an exterior surface of the third tube. In some embodiments, a gap may be provided between a distal most point of the first tube 302 and a distal most point of a fourth tube, which surrounds the third tube. The gap may be provided to allow receipt of the band(s) about the third tube upon deployment. The second tube of the band loader may then be biased relative to the first tube to deploy the band from the first tube to the third tube of the applicator.

In some embodiments, once the band has been deployed about the third tube, the method may include disengaging the band loader from the applicator. The third tube of the applicator may then be brought into position about the target tissue, and the band may be deployed by biasing the distal most point of the fourth tube against the band until the band is slid or pushed beyond a fourth tube distal end of the third tube and onto the target tissue.

In some embodiments, a physician may operate the medical device described herein by inserting the medical device into a rectum of a patient. Next, the physician may engage the target tissue within the patient with the medical device, and deploy one or more bands by biasing the outer tube relative to the inner tube. As a result, one or more bands are deployed onto the target tissue.

All directional references (e.g., proximal, distal, upper, lower, upward, downward, left, right, lateral, longitudinal, front, back, top, bottom, above, below, vertical, horizontal, radial, axial, clockwise, and counterclockwise) are only used for identification purposes to aid the reader's understanding of the present disclosure, and do not create limitations, particularly as to the position, orientation, or use of this disclosure. Although non-limiting, as used herein with respect to the device 100 and device 200, the term "proximal" may refer to a portion of the device(s) closest to an operator during use, while the term "distal" may refer to a portion of the device(s) first inserted within a patient.

Some embodiments may be described using the expression "coupled" and "connected" along with their derivatives. These terms are not intended as synonyms for each other. For example, some embodiments may be described using the terms "connected" and/or "coupled" to indicate that two or more elements are in direct physical or electrical contact with each other. The term "coupled," however, may also mean that two or more elements are not in direct contact with each other, but yet still co-operate or interact with each other.

As used herein, the singular forms "a," "an," and "the" are intended to include the plural forms as well, unless the context clearly indicates otherwise. It will be further understood that the terms "comprises" and/or "comprising," or "includes" and/or "including" when used herein, specify the presence of stated features, regions, steps elements and/or components, but do not preclude the presence or addition of one or more other features, regions, integers, steps, operations, elements, components and/or groups thereof.

Furthermore, the terms "substantial" or "substantially," as well as the terms "approximate" or "approximately," can be used interchangeably in some embodiments, and can be described using any relative measures acceptable by one of skill. For example, these terms can serve as a comparison to a reference parameter, to indicate a deviation that will still provide the intended function. Although non-limiting, the deviation from the reference parameter can be, for example, in an amount of less than 1%, less than 3%, less than 5%, less than 10%, less than 15%, less than 20%, and so on.

Although specific embodiments have been illustrated and described herein, it should be appreciated that any arrangement calculated to achieve the same purpose may be substituted for the specific embodiments shown. This disclosure is intended to cover any and all adaptations or variations of various embodiments. It is to be understood that the above description has been made in an illustrative fashion, and not a restrictive one. Combinations of the above embodiments, and other embodiments not specifically described herein will be apparent to those of skill in the art upon reviewing the above description. Thus, the scope of various embodiments includes any other applications in which the above compositions, structures, and methods are used.

Still furthermore, although the illustrative method 400 is described above as a series of acts or events, the present disclosure is not limited by the illustrated ordering of such acts or events unless specifically stated. For example, some acts may occur in different orders and/or concurrently with other acts or events apart from those illustrated and/or described herein, in accordance with the disclosure. In addition, not all illustrated acts or events may be required to implement a methodology in accordance with the present disclosure.

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

## Claims

1. A medical device (100, 200), comprising:
a first tube including a plurality of bands (130, 230) disposed on a first tube distal end (134, 234),
wherein each of the plurality of bands is disposed in one or more corresponding indentations (155, 255) of the first tube or wherein a plurality of protrusions extending outwardly from the first tube separates the plurality of bands; and
a second tube (104, 204) for receiving the first tube, the second tube comprising a second tube proximal end (143, 243) opposite a second tube distal end (146, 246), wherein the second tube distal end includes an engagement member (145, 245) operable to dispense the plurality of bands from the first tube in response to axial movement of the first and second tubes relative to one another,
**characterized in that** the medical device comprises a plunger (106, 206) movable within the first tube (102, 202),
wherein the plunger is configured to be biased to apply a suction to bring a target hemorrhoid tissue into the first tube.

2. The medical device of claim 1, the plunger (106, 206) comprising:
a shaft (116, 216) extending between a plunger proximal end (118, 218) and a plunger distal end (120, 220); and
a sealing member (124, 224) extending around the shaft, wherein the sealing member engages an inner surface of the first tube (102, 202).

3. The medical device of claim 2, the first tube (102, 202) comprising:
a first section (126, 226), wherein the plurality of bands (130, 230) extends around an exterior surface (131, 231) of the first section; and
a second section (127, 227) connected to the first section, wherein the second section includes a user engagement feature (136, 236), and wherein the sealing member is disposed within the second section.

4. The medical device of claim 3, wherein a first diameter (D1) of the first section (126, 226) is less than a second diameter (D2) of the second section (127, 227).

5. The medical device of any of claims 3-4, the second tube (104, 204) including a distal section (132, 232) connected with a proximal section (133, 233), wherein the proximal section extends around the second section (127, 227) of the first tube (102, 202), and wherein the distal section extends around the first section (126, 226) of the first tube.

6. The medical device of claim 5, the engagement member (145, 245) comprising:
a fixed end (147, 247) connected with the distal section (132, 232) of the second tube (104, 204); and
a free end (148, 248) extending axially from the fixed end, the free end comprising a flange (158, 258) extending radially towards the first tube (102, 202).

7. The medical device of claim 6, the flange (158, 258) comprising:
a sloped surface (161, 261) operable to pass over the plurality of bands (130, 230) as the first tube distal end (134, 234) and the second tube distal end (146, 246) move axially away to one another; and
a planar surface (164, 264) operable to engage the plurality of bands as the first tube distal end and the second tube distal end move axially towards one another.

8. The medical device of any of claims 6-7, the second tube (204) comprising a protection region (244) between the second tube distal end (246) and the engagement member (245).

9. The medical device of any of claims 6-8, wherein the free end (148, 248) terminates proximal of a distal most point (249) of the second tube distal end (146, 246).

10. The medical device of any of claims 1-9, further comprising one or more additional engagement members disposed about the second tube distal end (146, 246).

11. The medical device of claim 8, wherein the engagement member (245) and the one or more additional engagement members are separated from one another by a slot (252).

12. The medical device of claim 11, wherein the slot (252) extends axially along the distal section (232) of the second tube (204).

13. The medical device of claim 1, further comprising:
an applicator (301) engageable with a band loader (303), wherein the band loader comprises the first tube (302) and the second tube (304), and wherein the applicator comprises:
a third tube (380) surrounding the plunger; and
a fourth tube (381) surrounding the third tube.

14. The medical device of claim 13, wherein the third tube (380) is positionable within the first tube (302) to receive the plurality of bands (330) from the first tube (380).

15. The medical device of any of claims 13-14, wherein the fourth tube (381) includes a fourth tube distal end (396) operable to dislodge and advance the plurality of bands (330) from the third tube (380) as the third and fourth tubes move axially relative to one another.

## Patentansprüche

1. Medizinische Vorrichtung (100, 200), aufweisend:
ein erstes Rohr mit mehreren Bändern (130, 230), die an einem distalen Ende (134, 234) des ersten Rohrs angeordnet sind,
wobei jedes der mehreren Bänder in einer oder mehreren entsprechenden Vertiefungen (155, 255) des ersten Rohrs angeordnet ist, oder wobei mehrere sich vom ersten Rohr nach außen erstreckende Vorsprünge die mehreren Bänder voneinander trennen; und
ein zweites Rohr (104, 204) zum Aufnehmen des ersten Rohrs, wobei das zweite Rohr ein proximales Ende (143, 243) des zweiten Rohrs gegenüber einem distalen Ende (146, 246) des zweiten Rohrs aufweist, wobei das distale Ende des zweiten Rohrs ein Eingriffselement (145, 245) aufweist, das betätigbar ist, um die mehreren Bänder in Antwort auf eine axiale Bewegung des ersten und des zweiten Rohrs relativ zueinander vom ersten Rohr freizugeben,
**dadurch gekennzeichnet, dass**
die medizinische Vorrichtung einen Kolben (106, 206) aufweist, der innerhalb des ersten Rohrs (102, 202) bewegbar ist,
wobei der Kolben dafür konfiguriert ist, vorgespannt zu werden, um eine Saugkraft auszuüben, um ein Zielhämorrhoidalgewebe in das erste Rohr zu bringen.

2. Medizinische Vorrichtung nach Anspruch 1, wobei der Kolben (106, 206) aufweist:
einen sich zwischen einem proximalen Kolbenende (118, 218) und einem distalen Kolbenende (120, 220) erstreckenden Schaft (116, 216); und
ein sich um den Schaft herum erstreckendes Dichtungselement (124, 224), wobei das Dichtungselement mit einer Innenfläche des ersten Rohrs (102, 202) in Eingriff steht.

3. Medizinische Vorrichtung nach Anspruch 2, wobei das erste Rohr (102, 202) aufweist:
einen ersten Abschnitt (126, 226), wobei sich die mehreren Bänder (130, 230) um eine Außenfläche (131, 231) des ersten Abschnitts erstrecken; und
einen mit dem ersten Abschnitt verbundenen zweiten Abschnitt (127, 227), wobei der zweite Abschnitt ein Benutzereingriffsmerkmal (136, 236) aufweist, und wobei das Dichtungselement innerhalb des zweiten Abschnitts angeordnet ist.

4. Medizinische Vorrichtung nach Anspruch 3, wobei ein erster Durchmesser (D1) des ersten Abschnitts (126, 226) kleiner ist als ein zweiter Durchmesser (D2) des zweiten Abschnitts (127, 227).

5. Medizinische Vorrichtung nach Anspruch 3 oder 4, wobei das zweite Rohr (104, 204) einen distalen Abschnitt (132, 232) aufweist, der mit einem proximalen Abschnitt (133, 233) verbunden ist, wobei sich der proximale Abschnitt um den zweiten Abschnitt (127, 227) des ersten Rohrs (102, 202) erstreckt, und wobei sich der distale Abschnitt um den ersten Abschnitt (126, 226) des ersten Rohrs erstreckt.

6. Medizinische Vorrichtung nach Anspruch 5, wobei das Eingriffselement (145, 245) aufweist:
ein mit dem distalen Abschnitt (132, 232) des zweiten Rohrs (104, 204) verbundenes festes Ende (147, 247); und
ein sich vom festen Ende axial erstreckendes freies Ende (148, 248), wobei das freie Ende einen Flansch (158, 258) aufweist, der sich radial in Richtung zum ersten Rohr (102, 202) erstreckt.

7. Medizinische Vorrichtung nach Anspruch 6, wobei der Flansch (158, 258) aufweist:
eine geneigte Fläche (161, 261), die dazu eingerichtet ist, sich über die mehreren Bänder (130, 230) zu bewegen, wenn sich das distale Ende (134, 234) des ersten Rohrs und das distale Ende (146, 246) des zweiten Rohrs axial voneinander weg bewegen; und
eine ebene Fläche (164, 264), die dazu eingerichtet ist, mit den mehreren Bändern in Eingriff zu kommen, wenn sich das distale Ende des ersten Rohrs und das distale Ende des zweiten Rohrs axial zueinander hin bewegen.

8. Medizinische Vorrichtung nach Anspruch 6 oder 7, wobei das zweite Rohr (204) einen Schutzbereich (244) zwischen dem distalen Ende (246) des zweiten Rohrs und dem Eingriffselement (245) aufweist.

9. Medizinische Vorrichtung nach einem der Ansprüche 6 bis 8, wobei das freie Ende (148, 248) proximal von einem distalsten Punkt (249) des distalen Endes (146, 246) des zweiten Rohrs endet.

10. Medizinische Vorrichtung nach einem der Ansprüche 1 bis 9, ferner aufweisend ein oder mehrere zusätzliche Eingriffselemente, die um das distale Ende (146, 246) des zweiten Rohrs herum angeordnet sind.

11. Medizinische Vorrichtung nach Anspruch 8, wobei das Eingriffselement (245) und das eine oder die mehreren zusätzlichen Eingriffselemente durch einen Schlitz (252) voneinander getrennt sind.

12. Medizinische Vorrichtung nach Anspruch 11, wobei sich der Schlitz (252) axial entlang des distalen Abschnitts (232) des zweiten Rohrs (204) erstreckt.

13. Medizinische Vorrichtung nach Anspruch 1, ferner aufweisend:
einen Applikator (301), der mit einem Bandlader (303) in Eingriff bringbar ist, wobei der Bandlader das erste Rohr (302) und das zweite Rohr (304) aufweist, und wobei der Applikator aufweist:
ein den Kolben umgebendes drittes Rohr (380); und
ein das dritte Rohr umgebendes viertes Rohr (381).

14. Medizinische Vorrichtung nach Anspruch 13, wobei das dritte Rohr (380) innerhalb des ersten Rohrs (302) angeordnet werden kann, um die mehreren Bänder (330) vom ersten Rohr (380) aufzunehmen.

15. Medizinische Vorrichtung nach Anspruch 13 oder 14, wobei das vierte Rohr (381) ein distales Ende (396) des vierten Rohrs aufweist, das betätigbar ist, um die mehreren Bänder (330) vom dritten Rohr (380) zu verdrängen und vorwärts zu bewegen, wenn sich das dritte und das vierte Rohr axial relativ zueinander bewegen.

## Revendications

1. Dispositif médical (100, 200) comprenant :
un premier tube comprenant une pluralité de bandes (130, 230) disposées sur une extrémité distale (134, 234) du premier tube,
dans lequel chacune de la pluralité de bandes est disposée dans une ou plusieurs indentations (155, 255) correspondantes du premier tube ou dans lequel une pluralité de saillies s'étendant vers l'extérieur à partir du premier tube sépare la pluralité de bandes ; et
un deuxième tube (104, 204) pour recevoir le premier tube, le deuxième tube comprenant une extrémité proximale (143, 243) du deuxième tube opposée à l'extrémité distale (146, 246) du deuxième tube, dans lequel l'extrémité distale du deuxième tube comprend un élément de mise en prise (145, 245) pouvant fonctionner pour délivrer la pluralité de bandes du premier tube en réponse au mouvement axial des premier et deuxième tubes l'un par rapport à l'autre,
**caractérisé en ce que** le dispositif médical comprend un piston plongeur (106, 206) mobile dans le premier tube (102, 202),
dans lequel le piston plongeur est configuré pour être sollicité afin d'appliquer une aspiration pour amener un tissu hémorroïdaire cible dans le premier tube.

2. Dispositif médical selon la revendication 1, le piston plongeur (106, 206) comprenant :
un arbre (116, 216) s'étendant entre une extrémité proximale (118, 218) du piston plongeur et une extrémité distale (120, 220) du piston plongeur ; et
un élément d'étanchéité (124, 224) s'étendant autour de l'arbre, dans lequel l'élément d'étanchéité met en prise une surface interne du premier tube (102, 202).

3. Dispositif médical selon la revendication 2, le premier tube (102, 202) comprenant :
une première section (126, 226), dans lequel la pluralité de bandes (130, 230) s'étend autour d'une surface extérieure (131, 231) de la première section ; et
une deuxième section (127, 227) raccordée à la première section, dans lequel la deuxième section comprend une caractéristique de mise en prise d'utilisateur (136, 236) et dans lequel l'élément d'étanchéité est disposé dans la deuxième section.

4. Dispositif médical selon la revendication 3, dans lequel un premier diamètre (D1) de la première section (126, 226) est inférieur à un deuxième diamètre (D2) de la deuxième section (127, 227).

5. Dispositif médical selon l'une quelconque des revendications 3 à 4, le deuxième tube (104, 204) comprenant une section distale (132, 232) raccordée avec une section proximale (133, 233), dans lequel la section proximale s'étend autour de la deuxième section (127, 227) du premier tube (102, 202), et dans lequel la section distale s'étend autour de la première section (126, 226) du premier tube.

6. Dispositif médical selon la revendication 5, l'élément de mise en prise (145, 245) comprenant :
une extrémité fixe (147, 247) raccordée à la section distale (132, 232) du deuxième tube (104, 204) ; et
une extrémité libre (148, 248) s'étendant axialement à partir de l'extrémité fixe, l'extrémité libre comprenant une bride (158, 258) s'étendant radialement vers le premier tube (102, 202).

7. Dispositif médical selon la revendication 6, la bride (158, 258) comprenant :
une surface inclinée (161, 261) opérationnelle pour passer sur la pluralité de bandes (130, 230) lorsque l'extrémité distale (134, 234) du premier tube et l'extrémité distale (146, 246) du deuxième tube s'éloignent axialement l'une de l'autre ; et
une surface plane (164, 264) opérationnelle pour mettre en prise la pluralité de bandes lorsque l'extrémité distale du premier tube et l'extrémité distale du deuxième tube se rapprochent axialement l'une de l'autre.

8. Dispositif médical selon l'une quelconque des revendications 6 à 7, le deuxième tube (204) comprenant une région de protection (244) entre l'extrémité distale (246) du deuxième tube et l'élément de mise en prise (245).

9. Dispositif médical selon l'une quelconque des revendications 6 à 8, dans lequel l'extrémité libre (148, 248) se termine de manière proximale par rapport au point le plus distal (249) de l'extrémité distale (146, 246) du deuxième tube.

10. Dispositif médical selon l'une quelconque des revendications 1 à 9, comprenant en outre un ou plusieurs éléments de mise en prise supplémentaires disposés autour de l'extrémité distale (146, 246) du deuxième tube.

11. Dispositif médical selon la revendication 8, dans lequel l'élément de mise en prise (245) et les un ou plusieurs des éléments de mise en prise supplémentaires sont séparés les uns des autres par une fente (252).

12. Dispositif médical selon la revendication 11, dans lequel la fente (252) s'étend axialement le long de la section distale (232) du deuxième tube (204).

13. Dispositif médical selon la revendication 1, comprenant en outre :
un applicateur (301) pouvant se mettre en prise avec un chargeur de bande (303), dans lequel le chargeur de bande comprend :
le premier tube (302) et le deuxième tube (304), et dans lequel l'applicateur comprend :
un troisième tube (380) entourant le piston plongeur ; et
un quatrième tube (381) entourant le troisième tube.

14. Dispositif médical selon la revendication 13, dans lequel le troisième tube (380) peut être positionné dans le premier tube (302) pour recevoir la pluralité de bandes (330) du premier tube (380).

15. Dispositif médical selon l'une quelconque des revendications 13 à 14, dans lequel le quatrième tube (381) comprend une extrémité distale (396) du quatrième tube opérationnelle pour déloger et faire avancer la pluralité de bandes (330) du troisième tube (380), lorsque les troisième et quatrième tubes se déplacent de manière axiale l'un par rapport à l'autre.
